# FASCICULE DE BREVET EUROPEEN

(11) **EP 1 456 350 B1**
(45) Date de publication et mention de la délivrance du brevet: **07.02.2007**
(21) Numéro de dépôt: 02799423.5
(22) Date de dépôt: 18.09.2002
(51) Int. Cl.: C12N 1/20, A23B 4/22, C12R 1/46

(54) **BACTERIES LACTIQUES DU GENRE LACTOCOCCUS LACTIS ET LEUR APPLICATION A LA CONSERVATION DE PRODUITS ALIMENTAIRES**
MILCHSÄUREBAKTERIEN DER GATTUNG LACTOCOCCUS LACTIS UND DEREN VERWENDUNG ZUR KONSERVIERUNG VON NAHRUNGSMITTELN
LACTIC ACID BACTERIA OF THE GENUS LACTOCOCCUS LACTIS AND USE THEREOF FOR PRESERVING FOOD PRODUCTS

(30) Priorité: 21.09.2001 FR 0112187
(43) Date de publication de la demande: 15.09.2004
(73) Titulaire: Daniel, Patrice, 44130 Blain (FR); Lorre, Sylvie, 44100 Nantes (FR)
(72) Inventeur: Daniel, Patrice, 44130 Blain (FR); Lorre, Sylvie, 44100 Nantes (FR)
(74) Mandataire: Fosse, Danièle
(86) Numéro de dépôt international: PCT/FR2002/003180
(87) Numéro de publication internationale: WO 2003/027268

(56) Documents cités:
- WO-A-00/60947
- MAUGUIN S ET AL: "CHARACTERIZATION OF LACTIC ACID BACTERIA ISOLATED FROM SEAFOOD" JOURNAL OF APPLIED BACTERIOLOGY, OXFORD, GB, vol. 76, no. 6, 1994, pages 616-625, XP008020180
- CHEN MEI-HUI ET AL: "Processing of low-salted mackerel fillets using biopreservative." JOURNAL OF THE FISHERIES SOCIETY OF TAIWAN, vol. 24, no. 4, décembre 1997 (1997-12), pages 327-336, XP001077037 ISSN: 0379-4180 cité dans la demande
- FAR LACTIC ACID BACTERIA IN FISH PRESERVATION, [en ligne] 27 mars 2000 (2000-03-27), XP002199386 Extrait de l'Internet: <URL:http://exp.hispeed.com/flair/ffe14394 .htm> [extrait le 2002-05-13]
- WESSELS S ET AL: "SUITABILITY OF LACTOCOCCUS LACTIS SUBSP. LACTIS ATCC 11454 AS A PROTECTIVE CULTURE FOR LIGHTLY PRESERVED FISH PRODUCTS" FOOD MICROBIOLOGY, ACADEMIC PRESS LTD, LONDON, GB, vol. 13, no. 4, 1996, pages 323-332, XP000945352 ISSN: 0740-0020 cité dans la demande

## Description

La présente invention concerne une nouvelle souche de bactéries lactiques espèce Lactococcus lactis.

Cette invention se rapporte en outre à des cultures, biomasses, dérivées de cette souche et leur utilisation dans la conservation de produits alimentaires.

Cette invention se rapporte encore à des compositions incorporant une telle souche, à des produits alimentaires traités au moyen d'une telle souche et à des procédés de conservation de tels produits alimentaires.

La présente invention est le résultat d'études menées dans le domaine de la conservation de produits alimentaires, en particulier de produits de la mer conditionnés sous vide ou dans une atmosphère contrôlée.

Le poisson frais est un aliment particulièrement périssable rendant sa conservation difficile et limitant sa distribution. L'altération du poisson frais réfrigéré est provoquée par le développement de micro-organismes non pathogènes produisant de l'ammoniac, des amines, de l'hydrogène sulfuré... L'emballage sous vide ou sous atmosphère modifiée n'inhibe pas totalement cette flore d'altération dont une partie est capable de s'adapter à des conditions d'anaérobiose. Il en résulte le développement d'odeurs désagréables qui se concentrent rapidement, avant même que le goût du poisson ne soit altéré. Le "sous-vide" et l'atmosphère modifiée sont cependant des modes de conditionnement de plus en plus appréciés car ils allient les qualités organoleptiques et nutritionnelles du frais et une durée de conservation élevée.

L'utilisation de bactéries lactiques pour préserver les aliments réfrigérés a fait l'objet de différents brevets ou publications.

Ainsi, Boudreaux et al. en 1989 utilisent des cellules vivantes de Lactobacillus ayant la particularité de ne pas fermenter et capables de produire du peroxyde d'hydrogène pour inhiber la flore d'altération et la flore pathogène des aliments réfrigérés emballés (U.S. N° 4.874.704). Toutefois, le Peroxyde d'hydroxygène s'avère peu efficace pour les poissons et pose des problèmes en termes de réglementation.

Matrozza et al. (1989) rajoutent un mélange de Streptococcus lactis et de Pediococcus à base de cellules non viables pour inhiber la flore psychrotrophe du lait réfrigéré sans obtenir de phénomène de fermentation ou de croissance de la flore lactique (U.S. N° 4.880.743). Ce processus d'inhibition repose donc sur la production de molécules actives.

Dans le cadre d'un programme FAR en 2000, l'équipe du Dr Hall de l'Université de Loughborough a testé différentes souches de bactéries lactiques isolées de produits marins pour préserver de la chair hachée de merlan. L'apport de ferment (souches de Lactococcus ou de Carnobacterium) n'avait pas d'incidence sur les qualités organoleptiques des produits ni sur l'inhibition de la flore d'altération.

Hutkins et al. (1993) introduisent dans les aliments réfrigérés conservés en anaérobiose des cellules vivantes de Pediococcus productrices de bactériocines. Les bactéries lactiques rajoutées ne se développent pas dans l'aliment, ne le fermentent pas et ne modifient pas ses qualités organoleptiques (U.S. N° 5.186.962). Ces bactéries productrices de bactériocines s'avèrent non viables à long terme. En outre, les bactériocines sont considérées comme des additifs de type antibiotique provoquant l'apparition de germes résistants et pouvant perdre ainsi leur efficacité.

Des essais de préservation de saumon fumé à l'aide d'une souche de Lactococcus lactis productrice de nisine ont été réalisés par Wessels et Huss en 1996. Il s'avère que la population de Lactococcus lactis décroît sur le saumon fumé conservé à 10°C, la souche n'étant pas psychrotrophe, ni adaptée à ce type de substrat. L'action combinée du sel et du taux de CO₂ permet d'augmenter les effets bactéricides de la nisine contre des souches de Listeria monocytogènes (Nilsson et al. 1997).

Parallèlement, le même type de souche a été étudié par Chen Mei-Hui et Al. Des filets de maquereau ont été ensemencés par trempage dans une solution acide et salée contenant une forte concentration en cellules de Lactococcus lactis productrice de nisine. Après séchage, les filets sont conservés à 4°C dans des sacs. Les auteurs n'ont pas pu comparer l'évolution de la flore d'altération entre filets ensemencés et non ensemencés car les techniques de dénombrements classiques ne permettent pas de séparer la flore d'altération du ferment rajouté en très grande quantité (Chen Mei-Hui et Al.: "Processing of low-slated mackerel fillets using biopreservative". Journal of the Fisheries Society of Taiwan, vol. 24, no. 4, 1997 (1997-12), pages 327-336.

Des bactéries lactiques isolées de poisson, présentant des activités inhibitrices vis-à-vis de la flore d'altération du poisson ou de souches de Listeria monocytogènes ont fait l'objet de différentes études (Stoffels et al., 1992 ; Pilet et al., 1995; Leroi et al., 1996). Les souches étudiées sont toutes apparentées au genre Carnobactérium. Une souche de Leuconostoc isolée de poisson a également montré des aptitudes à inhiber des souches de Listeria monocytogènes (Jeppensen et Huss, 1993). Toutefois, ces souches poussent à 30°C. Il est donc impossible de séparer la flore d'altération de la souche au cours d'analyses microbiologiques de routine.

La demande internationale WO 00/60947 décrit de nouvelles souches de bactéries productrices d'acide lactique. Ces bactéries lactiques sont toutes isolées de produits laitiers et croissent à 30°C. Aucune des bactéries impliquées n'altère les aliments à des températures inférieures à 7°C parce qu'elles ne se développent pas à ces températures. La population bactérienne est divisée par 100 après une à deux semaines de conservation. Les inhibitions des germes pathogènes n'ont lieu qu'à des températures supérieures à 8°C. Elles sont dues à la présence d'acides, de dioxide de carbone et de bactériocines ou autres. En conséquence, ces bactéries lactiques présentent divers inconvénients puisqu'elles sont incapables d'inhiber des germes indésirables à des températures inférieures à 8°C sans produire de bactériocine et qu'elles se développent à 30°C.

Un but de la présente invention est de proposer une nouvelle souche de bactérie lactique apte à retarder le développement de mauvaises odeurs sur des produits alimentaires réfrigérés de préférence conditionnés dans une atmosphère contrôlée ou sous vide et à inhiber des germes proches ainsi que des germes pathogènes pour prolonger la durée de conservation (DLC) des produits alimentaires, en particulier les produits de la mer conditionnés.

Un autre but de l'invention est de proposer une nouvelle souche de bactérie lactique incapable de se développer à 30°C de manière à permettre le dénombrement de la flore d'altération de produits alimentaires dans le cadre d'analyses microbiologiques de routine.

A cet effet, la présente invention fournit une nouvelle souche de bactéries lactiques, à savoir Lactococcus lactis approprié pour une exploitation dans le domaine de la conservation des produits alimentaires, en particulier de produits de la mer.

Un échantillon de la culture du micro-organisme, conforme à l'invention, appelé souche Lactococcus lactis LLO, a été déposé le 11 septembre 2001 sous le Traité de Budapest dans la collection nationale de culture de micro-organismes française (CNCM), Institut Pasteur - 28 rue du Docteur Roux - 75724 PARIS CEDEX 15. L'échantillon a reçu le N° CNCM I-2716.

Conformément à cette invention, les cultures, biomasses obtenues à partir de la souche comme mentionnée ci-dessus sont également prévus. Bien évidemment, cette invention inclut les souches de Lactococcus lactis obtenues à partir de mutation, variation, recombinaison de la souche Lactococcus lactis LLO et présentant une température de croissance comprise entre 2°C et 25°C avec une température optimale de croissance proche de 20°C et une capacité de ralentissement des développement de la flore d'altération de produits alimentaires réfrigérés.

Un autre objet de l'invention concerne l'utilisation de souches cultures, biomasses, de Lactococcus lactis LLO pour la conservation de produits alimentaires en tant qu'agent retardant l'apparition de mauvaises odeurs ou ralentissant le développement de la flore d'altération de produits alimentaires réfrigérés et de préférence conditionnés dans une atmosphère réduite en oxygène.

Un autre objet de l'invention concerne une composition caractérisée en ce qu'elle contient au moins la souche Lactococcus lactis LLO ou un variant ou un mutant de celle-ci.

Un autre objet de l'invention concerne une composition alimentaire pour la conservation à l'état réfrigéré de produits alimentaires, en particulier de produits de la mer, conditionnés de préférence sous atmosphère réduite en oxygène, caractérisée en ce qu'elle comprend au moins Lactococcus lactis LLO ou un variant ou un mutant de celle-ci.

Un autre objet de l'invention concerne un produit alimentaire conservé à l'état réfrigéré et de préférence conditionné sous une atmosphère réduite en oxygène, caractérisé en ce que ledit produit présente à sa surface une barrière biologique constituée au moins de Lactococcus lactis LLO ou d'un variant ou d'un mutant de celui-ci.

Un autre objet de l'invention concerne un procédé de conservation à l'état réfrigéré de produits alimentaires, en particulier de produits de la mer, de préférence conditionnés dans une atmosphère réduite en oxygène avant consommation, caractérisé en ce qu'il consiste à mettre en contact le produit alimentaire à conserver et Lactococcus lactis préalablement au conditionnement dudit produit de manière à former une barrière biologique en surface dudit produit qui ralentit le développement de mauvaises odeurs et la croissance de la flore d'altération dudit produit.

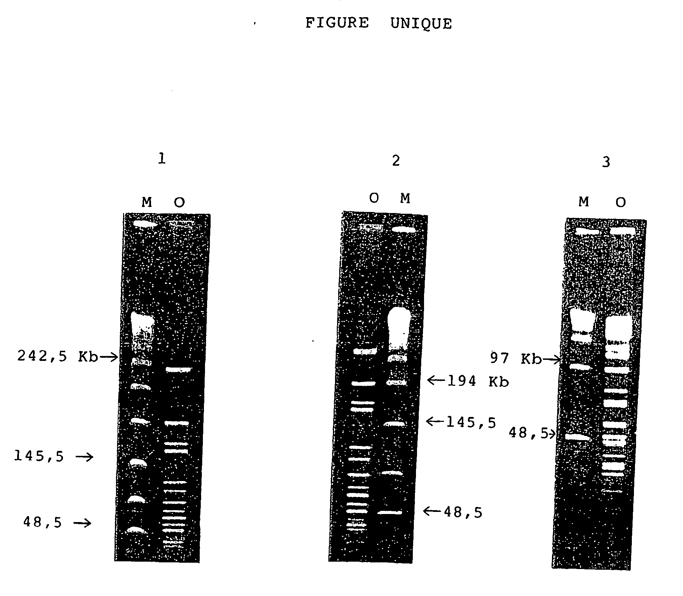

L'invention sera bien comprise à la lecture de la description suivante d'exemples de réalisation, en référence à la figure unique annexée qui représente, sous forme de trois colonnes, les migrations de différents fragments de restriction de l'ADN chromosomique de la souche LLO.

La souche Lactococcus lactis LLO a été isolée à partir de filets de merlan réfrigérés conservés en présence de teneur réduite en oxygène.

La souche Lactococcus lactis LLO correspond à la définition d'une bactérie lactique. Elle s'apparente au genre Lactococcus espèce Lactis. Son métabolisme dans le lait ou le fumet de poisson ne produit aucun phénomène d'altération. Elle inhibe la flore d'altération du merlan, du saumon et des crustacés.

Sur le plan morphologique, les cellules de la souche Lactococcus lactis LLO sont ovoïdes, immobiles. Elles apparaissent par paires ou en chaînes.

En ce qui concerne leurs caractéristiques de culture, ces cellules sont Gram positif, catalase négative, oxydase négative. Elles ne forment pas de spores. Elles sont anaérobies facultatives et ont un métabolisme fermentatif. La fermentation du glucose s'accompagne d'une légère production de gaz. La souche est cependant homofermentaire. Elle appartient au genre Lactococcus.

La souche n'hydrolyse pas l'arginine, l'urée ou l'amidon ne produit pas d'indole, ni d'hydrogène sulfuré et n'acidifie pas le lait. Elle ne réduit pas les nitrates, elle est α hémolytique. Elle produit de l'acétoïne.

La souche est psychrotrophe et se développe entre 2 et 25°C avec un optimum de croissance proche de 20°C. Elle se cultive dans les milieux de cultures suivants : MRS pH 6,5, Elliker, M17, gélose Trypticase Soja au sang. Elle ne pousse pas sur le milieu gélosé de ROGOSA, ni à 30°C. Elle se développe sur la chair de poissons ou de crustacés réfrigérée sans y apporter d'odeur, ni un goût altéré.

Un antibiogramme a été réalisé avec la souche LLO pour étudier sa résistance vis-à-vis de quelques antibiotiques.

Le test est réalisé sur milieu de Mueller Hinton contenant 1,7 % d'agar et 5 % de sang de cheval, le pH est de 7,3. L'épaisseur de la gélose dans les boîtes de Pétri est constante, elle est de 4 mm. Il s'agit d'un milieu standardisé selon les normes O.M.S.

La souche LLO est cultivée dans un bouillon MRS pH 6,5 pendant 48 à 36 heures à 15°C jusqu'à obtention de la phase stationnaire. La culture est diluée au 1/10^{ème} dans un bouillon Mueller Hinton. La concentration approximative de la suspension est de 10⁶ bactéries/ml.

Les milieux gélosés sont inondés entièrement avec 2 ml de la suspension. L'excès de liquide est retiré et les boîtes sont mises à sécher 10 minutes à température ambiante. Les disques antibiotiques sont déposés à l'aide d'une pince (4 maximum). Les boîtes sont incubées à température ambiante pendant 48 heures.

Les résultats sont tels que suit :

| Nom | Sigle | Famille | Résultat |
|---|---|---|---|
| Pénicilline G | P | Beta-lactamines | Sensible |
| Ampicilline | AM | | Sensible |
| Amoxicilline + Ac clavulanique | AMC | | Sensible |
| Céfalexine | CN | | Sensible |
| Streptomycine (10 U.I.) | S | Aminosides | Sensible |
| Kanamycine (30 U.I.) | K | | Sensible |
| Gentamicine (10 U.I.) | GM | | Sensible |
| Chloramphénicol | C | Phénicoles | Sensible |
| Tétracycline | TE | Tétracyclines | Sensible |
| Doxycycline | DO | | Sensible |
| Erythromycine | E | Macrolides | Sensible |
| Spiramycine | SP | | Sensible |
| Lincomycine | L | | Sensible |
| Clindamycine | CM | | Sensible |
| Bacitracine | B | Polypeptides | Sensible |
| Sulfamide triméthoprime | SXT | Sulfamides | Résistante |
| Fluméquine | UB | Quinolones | Résistante |
| Enrofloxacine | ENR | | Sensible |
| Furane | FT | Nitrofuranes | Résistante |

Une identification génétique a été réalisée par électrophorèse en champ pulsé après digestion du génome par une enzyme de restriction à sites rares.

Le mode opératoire est le suivant :

La biomasse bactérienne provenant de 10 ml de culture (48 heures en milieu MRS, 18°C) est récupérée par centrifugation (10 mn à 4000 g). Le culot cellulaire est repris dans 10 ml de tampon TE (tris 10 mM, EDTA 1 mM, pH 7,5) puis homogénéisé au vortex. La D0600 est ensuite prise. On centrifuge (10 mn à 4000 g) et on reprend le culot dans DO600/0,7 ml de tampon T100E (Tris 10 mM, EDTA 100 mM, pH 7,5). On mélange V/V la suspension cellulaire avec une gélose LMP (à bas point de fusion) en fusion (2 % dans TE). On laisse refroidir à 4°C et on découpe les plugs de 1 mm d'épaisseur. On incube 4 heures à 37°C dans 3 ml de tampon T100E contenant 2 mg/ml de lysozyme. On retire ensuite le mélange réactionnel. On incube 16 heures à 37°C dans 3 ml de tampon TESP (tris 10 mM; EDTA 0,5 M ; Sarkosyl 10 % pH 7,5) contenant 3 mg de pronase. Après incubation, on lave 6 fois les plugs avec 10 ml de tampon TE (30 mn chaque lavage) puis on laisse digérer 4 heures par l'enzyme de restriction dans les conditions suivantes: volume total 120 µl, 30U d'enzyme, 1,2 µl de BSA et 1/10 du tampon préconisé par le fabricant OZYME.

Après digestion, on lave le plug dans 10 ml de tampon TE (20 mn) puis on charge le gel de migration.

L'appareil utilisé est un GeneLine (Beckman).

L'enzyme utilisée est Apa I.

Trois conditions de migration sont appliquées afin de séparer dans les meilleures conditions les différents fragments de restriction obtenus. Les résultats sont fournis à la figure unique.

La première colonne correspond à 10 s de pulse pendant 16 heures pour identifier les gros fragments.

La deuxième colonne correspond à 7 s de pulse pendant 16 heures pour identifier les fragments intermédiaires.

La troisième colonne correspond à 2 s de pulse pendant 7 heures puis 4 s de pulse pendant 4 heures pour identifier les petits fragments.

Le tampon de migration est TBE 0,25 X et la température est de 12°C.

Les résultats sont représentés à la figure unique.

Les effets inhibiteurs de la souche LLO ont été recherchés en boîte de Pétri par la technique de double couches.

Les souches indicatrices ont été cultivées pendant 24 à 48 heures à 30°C en bouillon M17 ou MRS pour les bactéries lactiques et en bouillon nutritif pour les non lactiques, avant d'être ensemencées dans le même milieu contenant 1 % d'agar. La concentration finale en souche indicatrice est de l'ordre de 10⁷ CFU/ml. Cette gélose maintenue en surfusion à 46°C est versée en boîte de Pétri sur une gélose identique contenant 1,2 % d'agar préalablement refroidie. Après gélification de la deuxième couche, une goutte de culot de la souche "O" cultivée en bouillon M17 à 20°C pendant 3 jours et lavée dans l'eau physiologique est déposée sur chaque gélose. Les boîtes sont incubées à 30°C.

L'échelle de sensibilité des souches indicatives vis-à-vis de la souche LLO est établie telle que suit:
Absence de halo: -
Halo de 1 mm autour du dépôt : +
Halo de 2 à 3 mm autour du dépôt: ++

les résultats obtenus sont fournis dans le tableau ci-après:

| Souches indicatrices | Inhibition par la souche "O" |
|---|---|
| Lc. Lactis ATCC 11 454 | - |
| Staphylococcus spp. | ++ |
| Staphylococcus spp. | ++ |
| Staphylococcus spp. | ++ |
| Bacillus spp/ | - |
| Listeria inocua | + |
| Serratia proteamaculans | ++ |
| Leuconostoc mesenteroides | ++ |
| Pediococcus pentoceus | - |
| Pediococcus acidilactis | - |
| Lactobacillus plantarum | - |
| Lactobacillus farciminis | - |
| Lactobacillus casei | ++ |
| Lactobacillus brevis | - |
| Lactobacillus acidophilus | - |
| Carnobacterium piscicola | + |
| Carnobacterium divergens | - |

La souche LLO est inhibée par la souche Lactococcus lactis ATCC 11454 productrice de nisine.

Des expériences d'ensemencement de filets de merlan conservés en aérobie ont été réalisées avec la souche Lactococcus lactis LLO. Ces expériences sont décrites ci-dessous :

Des filets de merlan ont été achetés chez un poissonnier. Les filets témoins sont rangés dans un sac et conservés au réfrigérateur à 5°C. Les filets essais sont ensemencés avec la souche LLO par pulvérisation de la souche LLO en suspension dans l'eau physiologique avant d'être rangés dans un deuxième sac et conservés à 5°C. Sur ces deux types de filets sont effectuées des analyses sensorielles et des analyses microbiologiques. Les analyses sensorielles sont réalisées à partir d'un échantillon témoin et essai.

L'odeur et le goût après cuisson de chaque échantillon sont notés selon une échelle de notes telle que suit:

| | |
|---|---|
| 0 | putride |
| 1 - 2 | très mauvais |
| 3 - 4 | mauvais |
| 5 - 6 | acceptable |
| 7 - 8 | satisfaisant |
| 9 - 10 | très satisfaisant |

Les analyses microbiologiques sont effectuées notamment par dénombrements des germes mésophiles aérobies. Les dénombrements sont réalisés à partir d'un prélèvement de 10 9 de chair mis en suspension dans 90 ml d'eau physiologique stérile et homogénéisés pendant 3 minutes. Cette suspension est considérée comme la suspension mère.

Les dénombrements des germes mésophiles aérobies cultivant à 30°C en 3 jours sont réalisés à partir de dilutions au dixième de la suspension mère en utilisant la technique de dénombrement en profondeur. Le milieu de culture est la gélose PCA, gélose ordinaire pour le dénombrement en microbiologie alimentaire.

La souche LLO est dénombrée sur gélose MRS pH 6,5 en utilisant la technique de dénombrement en profondeur, les boîtes sont incubées 5 jours à 15°C.

Les résultats obtenus sont tels que suit:

En ce qui concerne les tests organoleptiques, le tableau ci-dessous fournit les résultats :

| Jours de stockage à 5°C | 0 | 2 | 4 |
|---|---|---|---|
| TEMOIN | 8/10 | 5/10 | 2/10 |
| ESSAI | 8/10 | 8/10 | 6/10 |

En ce qui concerne les dénombrements, le tableau ci-dessous reproduit les résultats obtenus :

| Jours de stockage à 5°C | TEMOIN (cfu/g) | ESSAI (cfu/g) | Souche LLO (cfu/g) |
|---|---|---|---|
| 0 | 13 000 | 13 000 | 7 600 000 |
| 2 | 460 000 | 53 000 | 7 100 000 |
| 4 | 1 800 000 | 340 000 | 8 700 000 |

Les expériences menées permettent de constater que les produits alimentaires ensemencés avec la souche Lactococcus lactis LLO sont toujours de meilleure qualité organoleptique que les témoins. On constate en particulier une forte diminution des odeurs d'ammoniac ou de H₂S permettant une augmentation de la durée de conservation de produits alimentaires traités. La consommation régulière de produits alimentaires ensemencés n'engendre aucun désordre digestif, ni phénomène d'allergie. On constate également que le développement de la flore banale est retardé.

Les résultats obtenus permettent donc de conclure à la possibilité d'utiliser une composition comprenant la souche Lactococcus lactis LLO ou un variant ou un mutant de celle-ci en tant que composition alimentaire pour la conservation à l'état réfrigéré de produits alimentaires. Par réfrigération, on entend des produits alimentaires conservés dans une plage de température comprise entre environ 0°C et 12°C. A l'inverse, on constate que cette souche ne se développe pas à une température de 30°C. Ceci permet un dénombrement de la flore des produits alimentaires dans le cadre d'analyses microbiologiques de routine.

On constate également que l'effet de cette souche est augmenté quand elle est appliquée sur des produits alimentaires conservés dans une atmosphère réduite en oxygène.

Des expériences ont ainsi été menées sur du saumon frais écossais (salmo salar), provenant d'une ferme d'élevage des îles Shetland, éviscéré et mis en glace pendant 4 jours avant traitement. Ce saumon est fileté avec la peau. Un des filets sert de témoin, l'autre d'essai.

En ce qui concerne la préparation d'un témoin, un filet de saumon frais témoin est tranché en huit portions de 100 à 200 g puis emballé dans des sacs individuels et mis sous vide avant d'être stocké dans une chambre froide entre 2 et 4°C.

L'échantillon essai est préparé de la même manière à l'exception du fait qu'il comporte entre l'étape de tranchage et l'étape d'emballage une étape d'ensemencement par pulvérisation avec la souche LLO en suspension dans l'eau physiologique.

Sur la base de ces deux échantillons sont effectués d'une part des analyses sensorielles, d'autre part des examens microbiologiques. Les analyses sensorielles sont réalisées à partir d'un échantillon témoin et essai. La couleur de la chair est contrôlée visuellement. Après ouverture des sachets, l'odeur est notée selon une échelle de notes. Si nécessaire, l'échantillon est cuit dans l'eau frémissante pendant quelques minutes avant d'être à nouveau noté.

L'échelle de notes s'établit telle que suit :

| | |
|---|---|
| 0 | putride |
| 1 - 2 | très mauvais |
| 3 - 4 | mauvais |
| 5 - 6 | acceptable |
| 7 - 8 | satisfaisant |
| 9 - 10 | très satisfaisant |

En ce qui concerne les examens microbiologiques, le pH est mesuré en différents points des échantillons. La valeur retenue est une moyenne de 3 mesures. Les dénombrements sont réalisés à partir d'un prélèvement de 10 g de chair mis en suspension dans 90 ml d'eau physiologique stérile et homogénéisés pendant 3 minutes. Cette suspension est considérée comme la suspension mère.

Les dénombrements des germes mésophiles aérobies cultivant à 30°C en 3 jours sont réalisés à partir de dilutions au dixième de la suspension mère en utilisant la technique de dénombrement en profondeur. Le milieu de culture est la gélose PCA, gélose ordinaire pour le dénombrement en microbiologie alimentaire.

La souche LLO est dénombrée sur gélose MRS pH 6,5 en utilisant la technique de dénombrement en profondeur, les boîtes sont incubées 5 jours à 15°C.

Les résultats obtenus sont tels que suit:

En ce qui concerne les tests organoleptiques, le tableau ci-dessous fournit les résultats :

| Jours de stockage depuis la pêche | 4 | 7 | 11 | 14 |
|---|---|---|---|---|
| TEMOIN | 10/10 | 8 et 9/10 | 4/10 | 1/10 |
| ESSAI | 10/10 | 9 et 10/10 | 7 et 8/10 | 5/10 |

En ce qui concerne les variations du pH, le tableau ci-dessous reproduit les résultats obtenus :

| Jours de stockage depuis la pêche | 4 | 7 | 11 | 14 |
|---|---|---|---|---|
| TEMOIN | 6,4 | 6,3 | 6,5 | 6,2 |
| ESSAI | 6,2 | 6,3 | 6,2 | 6,3 |

En ce qui concerne les dénombrements, le tableau ci-dessous fournit les résultats obtenus :

| Jours de stockage depuis la pêche | TEMOIN (cfu/g) | ESSAI (cfu/g) | Souche LLO (cfu/g) |
|---|---|---|---|
| 4 | 790 | 790 | 100 000 |
| 7 | 1 200 | 780 | 530 000 |
| 11 | 139 000 | 8 900 | 1 300 000 |
| 14 | 740 000 | 160 000 | 7 400 000 |

Les résultats montrent clairement qu'il n'existe aucune différence significative entre le pH des témoins et des produits traités. A l'inverse, les qualités organoleptiques des produits ainsi que le développement de la flore banale présentent des résultats positifs comparés aux échantillons témoins.

D'autres essais ont été menés sur des crevettes d'élevage étêtées et surgelées. Ces crevettes sont mises à décongeler dans l'eau tiède pendant 5 minutes, avant d'être cuites dans l'eau bouillante salée à 20 g/l pendant 1 minute. Après un rapide refroidissement sous l'eau courante, les crevettes sont plongées dans un bain glacé d'eau salée à 20 g/l.

Les crevettes "témoin" sont emballées par lot de dix dans des barquettes sous atmosphère modifiée. Les crevettes "essai" sont préalablement ensemencées avec la souche LLO par pulvérisation de la souche LLO en suspension dans l'eau physiologique. Elles sont ensuite emballées par lot de dix dans des barquettes sous atmosphère modifiée et conservées à 4°C.

Des analyses sensorielles sont réalisées à partir d'un échantillon "témoin" et "essai". La couleur de la chair est contrôlée visuellement. Après ouverture des sachets, le goût est noté selon une échelle de notes identique à celle fournie pour l'exemple du saumon ci-dessus.

Des dénombrements sont réalisés à partir d'un prélèvement de 10 g de crevettes mis en suspension dans 90 ml d'eau physiologique stérile et homogénéisés pendant 3 minutes. Cette suspension est considérée comme la suspension mère.

Les dénombrements des germes mésophiles, aérobies cultivant à 30°C en 3 jours, sont réalisés à partir de dilutions au dixième de la suspension mère en utilisant la technique de dénombrement en profondeur. Le milieu de culture est la gélose PCA, gélose ordinaire pour le dénombrement en microbiologie alimentaire.

La souche LLO est dénombrée sur gélose MRS pH 6,5 en utilisant la technique de dénombrement en profondeur, les boîtes sont incubées 5 jours à 15°C.

En ce qui concerne les tests organoleptiques, le tableau ci-dessous fournit les résultats :

| Jours de stockage à 4°C | 0 | 5 | 7 | 13 | 21 | 27 |
|---|---|---|---|---|---|---|
| TEMOIN | 10/10 | 8 et 9/10 | 8/10 | 7/10 | 7/10 | 6/10 |
| ESSAI | 10/10 | 9 et 10/10 | 9 et 10/10 | 9 et 10/10 | 9/10 | 9/10 |

En ce qui concerne les dénombrements, le tableau ci-dessous fournit les résultats obtenus :

| Jours de stockage à 4°C | TEMOIN (cfu/g) | ESSAI (cfu/g) | Souche LLO (cfu/g) |
|---|---|---|---|
| 0 | 16 000 | 16 000 | 5 600 000 |
| 7 | < 300 | < 300 | 3 800 000 |
| 13 | 72 000 | < 300 | 5 000 000 |
| 21 | 5 800 000 | < 300 | 6 400 000 |
| 27 | - | 450 | 7 300 000 |

Il est donc possible d'envisager une application industrielle de cette souche dans le cadre de procédé de conservation de produits alimentaires réfrigérés. Le procédé consistera alors à mettre en contact le produit alimentaire à conserver et la souche Lactococcus lactis LLO préalablement au conditionnement du produit de manière à former une barrière biologique en surface du produit qui ralentit le développement de mauvaises odeurs et la croissance de la flore d'altération du produit. Bien évidemment, cette mise en contact devra s'effectuer après tout traitement du produit, en particulier si ce dernier doit subir un traitement thermique. La mise en contact pourra s'effectuer par inoculation du produit alimentaire à conserver avec environ 10⁵ - 10⁷ cellules restant viables de Lactococcus lactis LLO par gramme de produit à conserver. Diverses méthodes d'inoculation peuvent être envisagées selon la forme de présentation de la souche. Ainsi, la souche Lactococcus lactis LLO peut être sous forme lyophilisée. Cette souche Lactococcus lactis LLO peut encore se présenter sous forme d'une suspension. L'ensemencement de l'aliment peut s'effectuer aussi par pulvérisation d'une poudre incorporant la souche Lactococcus lactis LLO, ou par pulvérisation d'une suspension cellulaire incorporant la souche précitée, ou par trempage de l'aliment dans un bain contenant la souche. La souche pourra être utilisée seule ou mélangée à un support.

Cette souche possède donc les propriétés:
- de se développer sur les produits de la mer, tels que poissons, crustacés ou autres, aux températures de réfrigération,
- de ne pas altérer l'aliment,
- de permettre, dans certaines conditions, de ralentir le développement de la flore banale d'altération de l'aliment et d'inhiber certains pathogènes,
- de retarder l'apparition d'odeurs indésirables, telles que des odeurs d'ammoniac ou de H2S.

Son efficacité est accrue quand l'aliment est emballé sous vide, sous atmosphère modifiée ou tout autre conditionnement réduisant la présence d'oxygène.

Elle ne se développe pas à 30°C et peut être dénombrée dans une analyse microbiologique alimentaire courante.

## Revendications

1. souche de Lactococcus lactis appelée souche Lactococcus lactis LLO et déposée à la CNCM sous le numéro d'accession CNCM I-2716.

2. Toute souche de Lactococcus lactis conforme à la revendication 1 ou obtenue par mutation, variation, recombinaison de la souche conforme à la revendication 1, **caractérisée en ce qu'**elle présente une température de croissance comprise entre 2°C et 25°C avec une température optimale de croissance proche de 20°C et une capacité de ralentissement du développement de la flore d'altération de produits alimentaires réfrigérés.

3. Cultures, biomasses obtenues à partir de souches conformes à l'une des revendications 1 et 2.

4. Composition,
**caractérisée en ce qu'**elle comprend au moins la souche Lactococcus lactis LLO ou un variant ou un mutant de celle-ci conformément aux revendications 1 et 2.

5. Composition selon la revendication 4,
**caractérisée en ce que** la souche Lactococcus lactis LLO est sous forme lyophilisée.

6. Composition selon la revendication 4,
**caractérisée en ce que** la souche Lactococcus lactis LLO est sous forme d'une suspension.

7. Composition alimentaire pour la conservation à l'état réfrigéré de produits alimentaires, en particulier de produits de la mer, conditionnés de préférence sous atmosphère réduite en oxygène,
**caractérisée en ce qu'**elle comprend la souche Lactococcus lactis LLO ou un variant ou un mutant de celle-ci selon l'une des revendications 1 et 2 notamment en tant qu'agent actif retardant l'apparition de mauvaises odeurs et/ou le développement de bactéries pathogènes ou non.

8. Produit alimentaire conservé à l'état réfrigéré et conditionné de préférence sous une atmosphère réduite en oxygène,
**caractérisé en ce que** ledit produit présente à sa surface une barrière biologique constituée au moins de Lactococcus lactis LLO ou d'un variant ou d'un mutant de celui-ci conformément à l'une des revendications 1 et 2.

9. Utilisation de souches, cultures, biomasses, de Lactococcus lactis LLO conformes à l'une des revendications 1 à 3 comme agent retardant l'apparition de mauvaises odeurs sur des produits alimentaires conservés à l'état réfrigéré et conditionnés de préférence sous une atmosphère réduite en oxygène.

10. Utilisation de souches, cultures, biomasses, de Lactococcus lactis LLO conformes à l'une des revendications 1 à 3 comme agent ralentissant le développement de la flore d'altération des produits alimentaires réfrigérés et conditionnés de préférence sous une atmosphère réduite en oxygène.

11. Procédé de conservation à l'état réfrigéré de produits alimentaires, en particulier de produits de la mer, conditionnés dans une atmosphère réduite en oxygène avant consommation,
**caractérisé en ce qu'**il consiste à mettre en contact le produit alimentaire à conserver et Lactococcus lactis LLO conforme à l'une des revendications 1 et 2 préalablement au conditionnement dudit produit de manière à former au moins une barrière biologique en surface dudit produit qui ralentit le développement de mauvaises odeurs et la croissance de la flore d'altération dudit produit.

12. Procédé selon la revendication 11,
**caractérisé en ce qu'**on met en contact le produit alimentaire à conserver et Lactococcus lactis LLO par inoculation du produit à conserver avec environ 10⁵ - 10⁷ cellules restant viables de Lactococcus, lactis LLO par gramme de produit à conserver.

## Claims

1. Strain of Lactococcus lactis called Lactococcus lactis LLO strain, deposited in the Collection Nationale de Cultures de Micro-organismes(CNCM) under accession number CNCM 1-2716.

2. Any strain of Lactococcus lactis according to Claim 1 or obtained by mutation, variation or recombination of the strain according to Claim 1, **characterized in that** it has a growing temperature of between 2°C and 25°C, with an optimum growing temperature of around 20°C, and a capacity for slowing down the development of flora that taints refrigerated food products.

3. Cultures and biomasses obtained from strains according to one of Claims 1 and 2.

4. Composition, **characterized in that** it comprises at least the Lactococcus lactis LLO strain or a variant or mutant thereof according to Claims 1 and 2.

5. Composition according to Claim 4, **characterized in that** the Lactococcus lactis LLO strain is in lyophilized form.

6. Composition according to Claim 4, **characterized in that** the Lactococcus lactis LLO strain is in the form of a suspension.

7. Edible composition for preserving food products, particularly seafood products, in the refrigerated state, said products preferably being packaged under an oxygen-reduced atmosphere, **characterized in that** it comprises the Lactococcus lactis LLO strain or a variant or mutant thereof according to one of Claims 1 and 2, especially as an active agent for delaying the appearance of bad odours and/or the development of pathogenic or non-pathogenic bacteria.

8. Food product preserved in the refrigerated state and preferably packaged under an oxygen-reduced atmosphere, **characterized in that** said product has on its surface a biological barrier consisting of at least Lactococcus lactis LLO or a variant or mutant thereof according to one of Claims 1 and 2.

9. Use of strains, cultures and biomasses of Lactococcus lactis LLO according to one of Claims 1 to 3 as an agent for delaying the appearance of bad odours on food products preserved in the refrigerated state and preferably packaged under an oxygen-reduced atmosphere.

10. Use of strains, cultures and biomasses of Lactococcus lactis LLO according to one of Claims 1 to 3 as an agent for slowing down the development of flora that taints refrigerated food products preferably packaged under an oxygen-reduced atmosphere.

11. Method of preserving food products, particularly seafood products, in the refrigerated state, said products being packaged under an oxygen-reduced atmosphere before consumption, **characterized in that** it consists in bringing the food product to be preserved into contact with Lactococcus lactis LLO according to one of Claims 1 and 2, prior to the packaging of said product, so as to form at least a biological barrier on the surface of said product for slowing down the development of bad odours and the growth of flora that taints said product.

12. Method according to Claim 11, **characterized in that** the food product to be preserved is brought into contact with Lactococcus lactis LLO by inoculation of the product to be preserved with about 10⁵ - 10⁷ viable cells of Lactococcus lactis LLO per gram of product to be preserved.

## Patentansprüche

1. Schicht von Lactococcus lactis, LLO-Lactococcus-lactis-Schicht genannt und bei der Collection Nationale de Cultures de Micro-organismes (CNCM) unter der Hinterlegungsnummer CNCM I-2716 hinterlegt.

2. Eine Schicht von Lactococcus lactis nach Anspruch 1 oder erzeugt durch Mutation, Variation, Rekombination der Schicht nach Anspruch 1, **dadurch gekennzeichnet, dass** sie eine Wachstumstemperatur von 2 °C bis 25 °C mit einer optimalen Wachstumstemperatur um 20 °C und eine Fähigkeit zur Verzögerung der Entwicklung der Verderbnisflora bei gefrorenen Nahrungsmitteln aufweist.

3. Kulturen, Biomassen, die ausgehend von Schichten nach einem der Ansprüche 1 und 2 erhalten werden.

4. Zusammensetzung, **dadurch gekennzeichnet, dass** sie mindestens die LLO-Lactococcus-lactis-Schicht oder eine Variante oder eine Mutante davon nach den Ansprüchen 1 und 2 umfasst.

5. Zusammensetzung nach Anspruch 4, **dadurch gekennzeichnet, dass** die LLO-Lactococcus-lactis-Schicht in gefriergetrockneter Form vorliegt.

6. Zusammensetzung nach Anspruch 4, **dadurch gekennzeichnet, dass** die LLO-Lactococcus-lactis-Schicht in Form einer Suspension vorliegt.

7. Zusammensetzung für die Konservierung von Nahrungsmitteln im gefrorenen Zustand, insbesondere von Meereserzeugnissen, die vorzugsweise in sauerstoffarmer Atmosphäre verpackt werden, **dadurch gekennzeichnet, dass** sie die LLO-Lactococcus-lactis-Schicht oder eine Variante oder eine Mutante davon nach einem der Ansprüche 1 und 2 umfasst, insbesondere als Wirkstoff, der das Auftreten schlechter Gerüche und/oder die Entwicklung pathogener oder nicht-pathogener Bakterien verzögert.

8. Nahrungsmittel, das im gefrorenen Zustand konserviert und vorzugsweise in sauerstoffarmer Atmosphäre verpackt wird, **dadurch gekennzeichnet, dass** dieses Nahrungsmittel an seiner Oberfläche eine biologische Barriere aufweist, die mindestens aus LLO Lactococcus lactis oder einer Variante oder einer Mutante davon nach einem der Ansprüche 1 und 2 besteht.

9. Verwendung von Schichten, Kulturen, Biomassen von LLO Lactococcus lactis nach einem der Ansprüche 1 bis 3 als Wirkstoff, der das Auftreten schlechter Gerüche an Nahrungsmitteln verzögert, die im gefrorenen Zustand konserviert und vorzugsweise in sauerstoffarmer Atmosphäre verpackt werden.

10. Verwendung von Schichten, Kulturen, Biomassen von LLO Lactococcus lactis nach einem der Ansprüche 1 bis 3 als Wirkstoff, der die Entwicklung der Verderbnisflora bei gefrorenen Nahrungsmitteln verzögert, die vorzugsweise in sauerstoffarmer Atmosphäre verpackt werden.

11. Verfahren zur Konservierung von Nahrungsmitteln im gefrorenen Zustand, insbesondere von Meereserzeugnissen, die vorzugsweise in sauerstoffarmer Atmosphäre verpackt werden, **dadurch gekennzeichnet, dass** das zu konservierende Nahrungsmittel und LLO Lactococcus lactis nach einem der Ansprüche 1 und 2 vor der Verpackung des Nahrungsmittel in Kontakt gebracht werden, sodass sich auf der Oberfläche des Nahrungsmittels mindestens eine biologische Barriere bildet, die das Auftreten schlechter Gerüche und das Wachstum der Verderbnisflora bei dem Nahrungsmittel verzögert.

12. Verfahren nach Anspruch 11, **dadurch gekennzeichnet, dass** das zu konservierende Nahrungsmittel und LLO Lactococcus lactis durch Inokulation des zu konservierenden Nahrungsmittels mit circa 10⁵ - 10⁷ lebensfähig gebliebenen Zellen von LLO Lactococcus lactis je Gramm des zu konservierenden Nahrungsmittels in Kontakt gebracht werden.
